# EUROPÄISCHE PATENTANMELDUNG

(11) **EP 0 853 952 A1**
(43) Veröffentlichungstag der Anmeldung: **22.07.1998**
(21) Anmeldenummer: 97101582.1
(22) Anmeldetag: 01.02.1997
(51) Int. Cl.: A61M 5/30, A61M 5/303, A61M 5/24

(54) **Transdermales Injektionssystem**

(30) Priorität: 17.01.1997 DE 19701494
(71) Anmelder: BOEHRINGER MANNHEIM GMBH, 68298 Mannheim (DE)
(72) Erfinder: Haar, Hans-Peter, Dr., 69168 Wiesloch (DE)

(57) **Zusammenfassung**

Die vorliegende Erfindung fällt in das Gebiet der Injektion von Flüssigkeiten in Gewebe durch Erzeugung eines Hochdruckstrahles, der die Haut zu durchdringen vermag. Sie betrifft ein transdermales Injektionssystem für Flüssigkeiten, beinhaltend ein erstes Behältnis, in dem sich die zu injizierende Flüssigkeit befindet und das zwei Bereiche besitzt, wobei der erste Bereich zusammendrückbar oder flexibel ist und der zweite Bereich mindestens eine Austrittsöffnung beinhaltet, durch die Flüssigkeit entweichen kann, ein zweites Behältnis, welches das erste Behältnis so umgibt, daß sich der zusammendrückbare Bereich zumindest teilweise innerhalb des zweiten Behältnisses befindet oder das zweite Behältnis so an das erste Behältnis angrenzt, daß der flexible Bereich durch eine Druckänderung im zweiten Behältnis deformiert werden kann, sowie einen aktivierbaren Gasgenerator, der sich innerhalb des zweiten Behältnisses befindet und der bei Aktivierung einen Gasdruck innerhalb des zweiten Gefäßes erzeugt, wodurch das erste Behältnis zusammengedrückt und Flüssigkeit durch die mindestens eine Austrittsöffnung aus dem ersten Behältnis herausgepreßt wird.

## Beschreibung

Die vorliegende Erfindung fällt in das Gebiet der Injektion von Flüssigkeiten in Gewebe durch Erzeugung eines Hochdruckstrahles, der die Haut zu durchdringen vermag.

Die vorliegende Erfindung betrifft ein transdermales Injektionssystem für Flüssigkeiten, beinhaltend
- ein erstes Behältnis, in dem sich die zu injizierende Flüssigkeit befindet und das zwei Bereiche besitzt, wobei der erste Bereich zusammendrückbar oder flexibel ist und der zweite Bereich mindestens eine Austrittsöffnung beinhaltet, durch die Flüssigkeit entweichen kann,
- ein zweites Behältnis, welches das erste Behältnis so umgibt, daß sich der zusammendrückbare Bereich zumindest teilweise innerhalb des zweiten Behältnisses befindet oder das zweite Behaltnis so an das erste Behältnis angrenzt, daß der flexible Bereich durch eine Druckänderung im zweiten Behältnis deformiert werden kann, sowie
- einen aktivierbaren Gasgenerator, der sich innerhalb des zweiten Behältnisses befindet und der bei Aktivierung einen Gasdruck innerhalb des zweiten Gefäßes erzeugt, wodurch das erste Behältnis zusammengedruckt und Flüssigkeit durch die mindestens eine Austrittsöffnung aus dem ersten Behältnis herausgepreßt wird.

Im Bereich der Medizin gibt es zahlreiche Methoden, Medikamente gezielt zu verabreichen. Es sind Darreichungsformen wie Tabletten, Zäpfchen, Cremes und dergleichen bekannt. Für eine Vielzahl von Wirkstoffen sind derartige Darreichungsformen jedoch ungeeignet, da sich die physiologisch aktiven Stoffe zersetzen, bevor sie wirksam werden können. Bei Tabletten zur oralen Einnahme muß der Arzneistoff z. B. derart beschaffen sein, daß er einerseits die aggressive Magensäure überdauert und andererseits durch die Magenwand oder die Darmwand in den Körperkreislauf aufgenommen werden kann. Für eine große Zahl von Medikamenten ist es nicht möglich, für den Patienten angenehme Darreichungsformen wie Tabletten oder Zäpfchen zu entwickeln. In diesen Fällen ist es daher notwendig, die Medikamente direkt in das Gewebe oder in die Blutbahn einzubringen. Es ist heute noch der Regelfall, daß die Injektion mit einer Spritze erfolgt. Im Stand der Technik sind jedoch auch schon seit langer Zeit Injektionsvorrichtungen bekannt, die ohne die fuhr den Patienten unangenehme Hohlnadel zur Injektion auskommen. Injektionssysteme ohne Hohlnadel verwenden einen Strahl von Flüssigkeit, der mit hoher Geschwindigkeit aus einer Düse austritt und das Gewebe zu durchdringen vermag. Eine derartige Injektion ist wesentlich schmerzärmer und kann auch von Personen ausgeführt werden, die nicht im Umgang mit einer Spritze geschult sind.

Mit einem Injektionssystem gemaß der vorliegenden Erfindung können prinzipiell alle in flüssiger Form verfügbaren Medikamente injiziert werden. Das Anwendungsgebiet umfaßt beispielsweise Schmerzmittel, Insulin und auch Proteinlösungen. Für Proteinlösungen, insbesondere Lösungen von Humanproteinen, hat es sich überraschenderweise herausgestellt, daß sie ohne Zerstörung per Hochdruckstrahl applizierbar sind.

Nadelfreie Injektionssysteme wurden bereits in den 50er und 60er Jahren beschrieben. In den US-Patenten 2,322,244 und 3,335,722 werden beispielsweise Vorrichtungen genannt, die mit Explosivstoffen zur Generierung des notwendigen Hochdruckstrahles arbeiten. Heutige Systeme, wie beispielsweise das Gerät Vitajet® er Firma Vitajet Corporation, verwenden zur Generierung des Hochdruckstrahles eine Stahlfeder, die vom Benutzer gespannt werden muß. Ein analog arbeitendes Gerät ist ebenfalls von der Firma Mediject Corporation im Markt. Derartige Systeme haben den Nachteil, daß der Benutzer eine Vielzahl relativ umständlicher Bedienungsschritte vornehmen muß. Zunächst wird die zu injizierende Flüssigkeit in das Injektionsgerät aufgezogen. Danach wird eine Stahlfeder gespannt, was über eine Drehung zweier Geräteteile gegeneinander erfolgt. Insbesondere für Personen, die krank oder wie viele Diabetiker manuell beeinträchtigt sind, bedeuten die erforderlichen Bedienschritte eine enorme Anstrengung. Eine Alternative hierzu bieten die bereits genannten Injektionssysteme mit Explosivstoffen, da die Energie aus dem Explosivstoffgezogen wird und kein umständliches Spannen von Federn erforderlich ist. Die Abkehr, die im Laufe der Zeit von derartigen Systemen stattgefunden hat, hängt mit gravierenden hygienischen und sicherheitstechnischen Nachteilen zusammen. In der US 3,335,722 wird ausgeführt, daß insbesondere die gegenseitige Kontamination von Explosivstoff und Medikament ein Problem derartiger Vorrichtungen ist. In der US-Patentschrift wird daher eine Anordnung vorgeschlagen, bei der Medikamentenkapsel und Explosivstoff getrennt vorliegen und eine spezielle Anordnung zur Energieübertragung verwendet wird. In der beschriebenen Vorrichtung wird ein Sprengsatz durch einen auftreffenden Stift gezündet, wie dies beispielsweise auch bei Gewehrkugeln der Fall ist. Das durch die Explosion entstehende Gas treibt einen Zylinder an, der mechanisch mit einem zweiten Zylinder verbunden ist, welcher einen Gummistopfen antreibt, der die in einer Ampulle befindliche Medikamentenflüssigkeit durch eine gegenüberliegende Düse herauspreßt. Zur Vermeidung der gegenseitigen Kontamination von Verbrennungsgasen und Medikament wird hier ein hoher technischer Aufwand benötigt, der die Injektionsvorrichtung verkompliziert und daher auch verteuert. Darüber hinaus wird eine Ampulle benötigt, in der ein Gummistopfen verschoben wird. Aufgrund der durch Verschiebung des Stopfens erzeugten Drücke in der Ampulle ist es notwendig, Vorkehrungen zu treffen, um Undichtigkeiten zwischen Ampulle und Stopfen zu vermeiden. Hierbei ist insbesondere die Materialauswahl der Ampulle kritisch, da eine Verformung bei Druckbeaufschlagung nicht zu Undichtigkeiten im Bereich des Stopfens führen darf Die im Stand der Technik bekannten Vorrichtungen weisen allgemein den Nachteil auf, daß zur Druckgenerierung gegeneinander verschiebbare Teile, zumeist Stopfen, verwendet werden und somit Dichtflächen kontrolliert werden müssen.

Aufgabe der vorliegenden Erfindung war es, eine einfache, kostengünstige und dennoch zuverlässige Vorrichtung zur nadelfreien Injektion von Flüssigkeit zur Verfügung zu stellen. Insbesondere war es Aufgabe der vorliegenden Erfindung, die gegenseitige Kontamination von Explosionsgas und Medikament mit einfachen Mitteln sicher zu vermeiden. Weiterhin war es Aufgabe der vorliegenden Erfindung, eine Injektionsvorrichtung zur Verfügung zu stellen, die vom Benutzer mit minimalem Aufwand bedient werden kann und die sowohl einfach im Aufbau als auch kostengünstig ist. Diese Aufgaben werden erfindungsgemäß durch ein System gemäß Anspruch 1 gelöst.

Bei dem Injektionssystem der vorliegenden Erfindung werden keine gegeneinander verschiebbaren Teile verwendet, und es werden Dichtflächen zwischen beweglichen Teilen vermieden. Statt dessen wird ein Behältnis verwendet, das einen Bereich besitzt, der zusammengedruckt werden kann oder der flexibel ist und daher deformiert werden kann. Die Verwendung eines derartigen Behältnisses hat den Vorteil, daß keine gegeneinander verschiebbare Teile notwendig sind, und Dichtflächen vermieden werden. Für ein Zusammendrucken des ersten Behältnisses befindet sich dieses in einem zweiten Behältnis, welches das erste Behältnis in seinem zusammendruckbaren Bereich zumindest teilweise umgibt oder das erste Behaltnis grenzt mit seinem flexiblen Bereich so an das zweite Behältnis an, daß eine Druckänderung im zweiten Behältnis zu einer Deformation des flexiblen Bereiches führt. Bei Aufbau eines Druckes innerhalb des zweiten Behältnisses wird im ersten Behältnis ebenfalls ein Druck erzeugt, wodurch die Flüssigkeit durch die Austrittsöffnung herausgedrückt wird.

Das erste Behaltnis des erfindungsgemäßen Systems besitzt einen ersten Bereich, der entweder zusammendrückbar gestaltet ist oder der flexibel ist, so daß er deformiert werden kann. Das erste Behältnis ist so gestaltet, daß durch Zusammendrucken oder Deformation des ersten Bereiches eine Druckänderung im Gefäßinneren erzeugt werden kann. Der erste Bereich ist vorzugsweise aus leicht deformierbaren Materialien aufgebaut, wie beispielsweise Kunststoffen oder Metallfolien. Zur Erzielung des erfindungsgemäßen Effektes ist es wichtig, daß beim Zusammendrücken oder bei der Deformation keine Teile mechanisch gegeneinander verschoben werden, so daß Reibflächen entstehen, deren Abdichtung schwer beherrschbar ist. Die Gefaßhülle im ersten Bereich bleibt während des Zusammendrückens bzw. der Deformation geschlossen, was aufgrund der Materialelastizität im ersten Bereich möglich ist. Die Deformation kann beispielsweise in dem Eindrücken eines Teiles einer Wandung bestehen, wobei die Dehnbarkeit des Wandmaterials genutzt wird, um sicherzustellen, daß die Gefäßhülle geschlossen bleibt. Dies ist jedoch mit einer relativ starken mechanischen Belastung der Wand verbunden, die durch Ausführungsformen reduziert werden kann, bei denen eine Kompression des Wandmaterials erfolgt oder die Wandung von einer konvexen Form in eine konkave Form bewegt wird. Bei letztgenannter Ausführungsform ist die Oberfläche des ersten Bereiches in beiden Formen im wesentlichen gleich, so daß starke Expansionen oder Kompressionen des Wandmaterials vermieden werden.

Durch die vorstehend genannten Ausführungsformen werden Scherungen und starke Dehnungen des Wandmaterials des ersten Gefäßes weitestgehend vermieden. Daher sind im Regelfall an das Wandmaterial lediglich geringe Forderungen bezüglich seiner mechanischen Eigenschaften zu stellen. Kunststoffe, wie Polyethylen oder Polypropylen, sind beispielsweise geeignet, wobei Wandstärken unterhalb eines Millimeters realisiert werden können. Besonders geeignete Wandstärken liegen im Bereich von 100 bis 200 µm. Metallfolien können ebenfalls in dem genannten Dickenbereich eingesetzt werden.

Das erste Gefäß besitzt weiterhin einen zweiten Bereich, in dem sich eine Austrittsöffnung für Flüssigkeiten befindet. Hierzu liegt ein Austrittskanal in der Wandung des zweiten Bereiches, der in die Austrittsöffnung mündet. Der zweite Bereich ist weiterhin mechanisch so stabil, daß keine wesentliche Deformation durch den im ersten Behältnis entstehenden Druck erfolgt. Insbesondere sollte durch eine geeignete geometrische Anordnung verhindert werden, daß der Austrittskanal stark deformiert werden kann. Geeignete Durchmesser der Austrittsöffnung sind dem Fachmann aus dem Stand der Technik bekannt. Bei Verwendung von Explosivstoffen können jedoch sehr hohe Drücke erzeugt werden, so daß kleinere Austrittsöffnungen zugänglich werden, die im Bereich von 80 bis 130 µm liegen. An die geometrische Gestaltung von Austrittskanal und Austrittsöffnung sind keine besonderen Anforderungen zu stellen. Vorteilhaft ist jedoch eine Ausgestaltung, mit der ein fokussierter Flüssigkeitsstrahl erzeugt wird.

Aus hygienischen Gründen ist es normalerweise notwendig, die Austrittsöffnung zu verschließen, um ein Auslaufen und eine Kontamination der Flüssigkeit im ersten Behältnis zu verhindern. Vorzugsweise wird ein solcher Verschluß durch einen Zapfen oder dergleichen realisiert, der mit dem zweiten Bereich über eine Sollbruchstelle verbunden ist. Weiterhin können Schraubkappen, Aufsteckkappen usw. als Verschluß dienen.

Erster und zweiter Bereich des ersten Behältnisses sind vorteilhaft als eine Einheit ausgebildet. Insbesondere kann das erste Behältnis einteilig hergestellt werden, wie dies für Augentropfenampullen bekannt ist. In einem Spritzgußprozeß wird zunächst der zweite Bereich, beinhaltend den Austrittskanal, geformt, an den sich ein offener Kunststoffmantel anschließt, der später den zweiten Bereich bildet. Es wird Flüssigkeit in den Kunststoffmantel eingefüllt und die Öffnung durch Verschweißen des Mantelmaterials verschlossen.

Das erfindungsgemäße System wird vorteilhaft so gestaltet, daß der Prozeß des Zusammendrückens bzw. der Deformation nur im ersten Bereich erfolgt und der zweite Bereich keiner Deformation unterliegt. Ebenfalls der Übrgang von erstem und zweitem Bereich sollte nach Möglichkeit nur wenig deformiert werden.

Bei einer vorteilhaften Ausgestaltung befindet sich der zusammendruckbare Teil des ersten Behältnisses vollständig innerhalb des zweiten Behältnisses. Es ist weiterhin günstig, wenn das zweite Behältnis durch das erste Behältnis und ggf. weitere Materialteile vollständig verschlossen ist, so daß kein Gas austreten kann, selbst wenn der Innenraum einen weit höheren Druck aufweist als der Außenraum. Bei sehr schneller Generierung des Druckes, beispielsweise durch einen schnell abbrennenden Explosivstoff, ist es nicht unbedingt notwendig, daß das zweite Behältnis gegenüber dem Außenraum vollständig verschlossen ist. Es können vielmehr auch kleine Öffnungen vorgesehen werden, durch die Gas entweichen kann. Bei einer sehr schnellen Gasgenerierung ist der Druckaufbau im zweiten Behältnis so schnell, daß hierdurch während des Ausstoßprozesses kein signifikanter Druckabfall erfolgt. Wie erfindungsgemäß vorgesehen findet ein Zusammendrücken des ersten Behältnisses statt. Erst langsam wird der im zweiten Behältnis entstandene Druck durch Entweichen von Gas abgebaut. Eine derartige Ausführungsform des Injektionssystems kann dann vorteilhaft sein, wenn das Verbrennungsgas einen großen Anteil bei Raumtemperatur nicht kondensierender Bestandteile enthält, da in einem solchen Fall nach Verwendung des Injektionssystems ein hoher Druck im zweiten Gefäß bestehen bleiben wurde. Dies ist im Normalfall nicht nachteilig, kann jedoch bei Ausführungsformen mit einem dünnwandigen zweiten Behältnis zu einer Deformation dieses Behältnisses führen, die vom Benutzer als beunruhigend empfünden wird. In der Mehrzahl der Fälle von Gasgeneratoren, insbesondere bei Verwendung von Explosivstoffen, ist der Anteil an bei Raumtemperatur kondensierenden Substanzen jedoch so groß, daß nach Verwendung des Injektionssystems kein wesentlicher Überdruck im zweiten Behaltnis bestehen bleibt. Es kann daher im Normalfall auf Öffnungen im zweiten Behältnis verzichtet werden.

Ein erfindungsgemäßes Injektionssystem beinhaltet weiterhin einen aktivierbaren Gasgenerator. Als Gasgeneratoren kommen beispielsweise Explosivstoffe, wie Schwarzpulver, Nitrozellulose, Pentaerythrittetranitrat und dergleichen in Frage. Es sind insbesondere Explosivstoffe von Vorteil, die keine Schwermetalle, wie z. B. Blei oder Quecksilber, enthalten, da sie eine Umweltgefährdung vermeiden. Weiterhin werden vorteilhaft Explosivstoffe eingesetzt, die bei Explosion vollständig in Kohlendioxid und Wasser zerfallen.

Durch die Auswahl des Explosivstoffes und dessen geometrischer Anordnung kann die Explosions- bzw. Abbrennkinetik gesteuert werden. Im Rahmen der vorliegenden Erfindung ist es vorteilhaft, wenn der Explosivstoff nicht explosiv abbrennt, d. h. daß die durch die Verbrennung erzeugte Druckwelle eine Geschwindigkeit unterhalb der Schallgeschwindigkeit aufweist. Vorteilhaft ist es insbesondere, wenn der gesamte Abbrennvorgang des Explosivstoffes im Bereich von 10 bis 20 msec liegt. Um dies zu erreichen, wird der Explosivstoff in der Regel hochverdichtet, um ein Voranschreiten der Brennzone zu verlangsamen. Ein weiterer Faktor, der die Kinetik des Druckanstieges im zweiten Behältnis beeinflußt, ist die Größe des Hohlraumes im zweiten Behältnis, der vor der Aktivierung des Gasgenerators bereits mit Gas angefüllt ist. Je größer dieser Gasraum im zweiten Behaltnis ist, desto langsamer erfolgt der Druckaufbau.

Als Gasgenerator können auch andere Vorrichtungen dienen, die zum Aufbau eines Druckes im zweiten Behältnis geeignet sind. Dies kann beispielsweise ein weiteres Behältnis mit stark komprimiertem Gas oder auch unter Druck verflüssigtem Gas (wie beispielsweise CO₂ in einem Trägermaterial) sein. Das komprimierte Gas kann einerseits werkseitig abgefüllt sein, wie dies beispielsweise von CO₂-Patronen für Siphons bekannt ist, oder das Gas kann vom Benutzer selbst komprimiert werden, wie dies bei den im Handel befindlichen transdermalen Injektionssystemen (Mediject, Vitajet) der Fall ist. Bei diesen Vorrichtungen wird vom Benutzer eine Feder gespannt, die auf den Kolben eines Zylinders drückt und das im Zylinder befindliche Gas komprimiert. Bei einer solchen Ausführungsform könnte beispielsweise das zweite Behältnis des erfindungsgemäßen Systems als Zylinder ausgebildet sein, in dem ein Kolben läuft, der über eine Feder angetrieben wird. Bei einer derartigen Vorrichtung kann die Druckgenerierung durch Auslösung der Feder gesteuert werden. Bei Systemen, die mit werkseitig komprimierten Gasen arbeiten, kann die Freisetzung beispielsweise mit Durchstechen einer Versiegelung des Druckbehältnisses erfolgen. Ein solcher Prozeß wird beispielsweise auch bei CO₂-Siphons angewendet, bei denen eine CO₂-Patrone vom Benutzer auf eine spitze Hohlnadel aufgeschraubt wird, wodurch eine Metallfolie der CO₂-Patrone geöffnet wird und das CO₂-Gas durch die Hohlnadel ausströmt.

Der Gasgenerator kann weiterhin durch schnelle Verdampfung einer Flüssigkeit, beispielsweise mit einer elektrischen Heizwendel oder durch die elektrische Zerlegung eines Stoffes (meist einer Flüssigkeit) in Gase, erfolgen. Ein Beispiel für den letzteren Prozeß ist die Elektrolyse einer wäßrigen Lösung, bei der gasförmige Produkte, in der Regel Wasserstoff und Sauerstoff entstehen. Weiterhin können auch chemische Prozesse zur Gasgenerierung verwendet werden, so z. B. die Reaktion von fein verteiltem Aluminium mit Natronlauge, durch die Wasserstofffreigesetzt wird.

Ein transdermales Injektionssystem besitzt eine Austrittsöffnung durch die Flüssigkeit aus dem ersten Behältnis entweichen kann. Vorzugsweise dient die am ersten Behältnis befindliche Austrittsöffnung als Düse, durch die ein Fluidstrahl direkt durch die Haut injiziert wird. Es sind jedoch auch Ausführungsformen möglich, bei denen die Austrittsöffnung in eine Düse einmündet, durch die die Flüssigkeit injiziert wird. Dies ist beispielsweise möglich, indem das erste Behältnis mit seinem zweiten Bereich, in dem sich die Austrittsöffnung befindet, an eine Wandung angepreßt wird, in der sich die Düse befindet, so daß Austrittsöffnung und Düse einen fortlaufenden Kanal bilden. Die Injektion der Flüssigkeit mit erfindungsgemäßem System kann sowohl über eine Austrittsöffnung als auch über eine Düse erfolgen. Der Begriff Düse impliziert lediglich, daß der Kanal, durch den die Flüssigkeit aus dem ersten Behaltnis austritt, eine Formgebung besitzt, durch die die Geometrie des austretenden Flüssigkeitsstrahles gesteuert werden kann. Es hat sich jedoch herausgestellt, daß die Geometrie des austretenden Strahles von relativ geringer Bedeutung für den Erfolg des Injektionsvorganges ist. Es kann jedoch über eine Fokussierung des Strahls erreicht werden, daß die Flüssigkeit in tiefere Gewebeschichten eindringt, während durch die Generierung eines diffusen Strahles eine Injektion in obere Gewebeschichten erfolgt. Als wichtig hat es sich jedoch herausgestellt, daß die Düse bzw. Austrittsöffnung direkt auf die Hautoberfläche aufgesetzt wird, da die Flüssigkeit einen Druckabfall erfährt, wenn sie durch eine Luftschicht hindurchtreten muß, so daß u. U. der Druck nicht mehr ausreicht, um die Hautoberfläche zu durchdringen. Sofern der Flüssigkeitsstrahl jedoch durch die Düse bzw. den Austrittskanal fokussiert wird, kann ein Abstand zwischen Austrittsöffnung und Hautoberfläche bis zu mehreren Millimetern toleriert werden.

In Fallen, bei denen die Flüssigkeit in hoher Konzentrierung zu unerwünschten Irritationen des Gewebes führt, können statt einer Austrittsöffnung zwei oder mehr Austrittsöffnungen für die zu injizierende Flüssigkeit vorgesehen werden. Hierdurch wird erreicht, daß die Flüssigkeit über ein größeres Gewebeareal verteilt wird und die lokalen Konzentrationen geringer gehalten werden können.

Die dem Stand der Technik bekannten Vorrichtungen, die mit Stahifedern zur Komprimierung eines Gases oder einem werkseitig hochverdichteten Gas arbeiten, besitzen den Nachteil, daß die auf diese Weise generierbaren Drücke relativ gering sind und demgemäß Düsen verwendet werden müssen, deren Querschnitt zwischen 130 und 200 µm liegt. Mit den erfindungsgemäß bevorzugten Ausführungsformen, die einen Explosivstoff einsetzen, können hingegen wesentlich höhere Drücke erzeugt werden, so daß auch Düsen mit Querschnitten unterhalb 130 µm zugänglich sind. Insbesondere sind Düsen bzw. Austrittsöffnungen günstig, deren Querschnitt zwischen 80 µm und 130 µm liegt, da mit Düsen in diesem Größenbereich eine sehr effiziente Injektion vorgenommen werden kann.

Ein besonderer Vorteil der vorliegenden Erfindung liegt darin, daß das Injektionssystem sehr kompakt und benutzerfreundlich gestaltet werden kann. Dies liegt zum einen daran, daß bei Verwendung von Explosivstoffen als Gasgenerator der Raumbedarf für den Gasgenerator sehr klein ist und auch die Mittel zur Aktivierung des Gasgenerators sehr einfach gestaltet sein können. Weiterhin ist es mit dem System der vorliegenden Erfindung möglich, wegwerfbare Module in den Handel zu bringen, die das erste Behältnis, das zweite Behältnis sowie den Gasgenerator umfassen. Ein solches Model braucht von dem Benutzer lediglich in eine Handhabungsvorrichtung eingesetzt zu werden, die eine Vorrichtung zur Aktivierung des Gasgenerators beinhaltet. Es ist sogar möglich, ein wegwerfbares Injektionssystem zur Verfügung zu stellen, das neben einem ersten Behältnis, einem zweiten Behältnis und einem aktivierbaren Gasgenerator auch noch eine Vorrichtung zur Aktivierung des Gasgenerators beinhaltet. Ein solches System kann beispielsweise mit einem Explosivstoff als Gasgenerator realisiert werden, wobei der Explosivstoff über einen Reibzünder zur Explosion gebracht wird. Vorteilhaft kann zur Aktivierung des Gasgenerators auch ein Piezo-Zünder verwendet werden, wie er beispielsweise von Feuerzeugen bekannt ist.

Der vorstehend genannte Vorteil, wegwerfbare Module in den Handel bringen zu können, liegt insbesondere darin begründet, daß der Gasgenerator mit in das Modul integriert werden kann, während er bei Systemen des Standes der Technik zur Handhabungseinheit gehört. Als Gasgeneratoren für ein solches wegwenbares Modul sind insbesondere solche geeignet, in denen die Energie zur Druckerzeugung bereits in gespeicherter Form vorliegt und nicht vom Benutzer, beispielsweise durch Aufziehen einer Feder, erzeugt werden muß.

Mit der vorliegenden Erfindung ist es auch möglich, Systeme zur Verfügung zu stellen, die zwei- oder mehrteilig ausgeführt sind. Bei derartigen Systemen wird das erste Behältnis vom Benutzer in das zweite Behältnis eingesetzt und die Anordnung so verschlossen, daß ein im zweiten Gefäß entstehendes Gas nicht entweichen kann, sondern einen Druck auf den zusammendrückbaren Bereich des ersten Gefäßes ausübt. Bei einer solchen Ausführungsform gehört das zweite Behältnis in der Regel zu einer Handhabungseinheit und die Handhabungseinheit besitzt eine Vorrichtung, mit der die Anordnung aus erstem und zweitem Behältnis so miteinander verschlossen werden kann, daß aus dem zweiten Gefaß während des Injektionsvorganges kein Gas entweichen kann. Das zweite Behältnis kann in einer solchen Ausführungsform beispielsweise ein Hohlzylinder sein, bei dem eine Stirnfläche geöffnet ist, durch die das erste Behältnis eingeführt werden kann. Der Zylinder ist im Bereich der offenen Stirnseite mit einer Verschlußvorrichtung versehen, die zur Einsetzung des ersten Behältnisses geöffnet wird und darauffolgend so verschlossen werden kann, daß sie das erste Behältnis vorzugsweise im zweiten Bereich dichtend umfaßt.

Bei einer derartigen zweiteiligen Ausführungsform des Injektionssystems können eine Vielzahl von Gasgeneratoren verwendet werden, wie sie weiter oben beschrieben wurden. Dies sind beispielsweise Explosivstoffkapseln, die vom Benutzer vor der Injektion in das zweite Behältnis eingesetzt werden, oder es können Gasgeneratoren verwendet werden, die mit einem Kolben arbeiten, der seinerseits durch eine komprimierte Feder angetrieben wird. All diese Ausführungsformen weisen gegenüber dem Stand der Technik den Vorteil auf, daß das erste Behältnis keine gegeneinander verschlebbaren Teile, wie beispielsweise einen Kolben, der in einem Zylinder läuft, aufweist. Dies ermöglicht sowohl eine kostengünstigere Herstellung des ersten Behältnisses als auch eine Gewährleistung, daß keine Vermischung von Gas mit der zu injizierenden Flüssigkeit erfolgt.

Die vorliegende Erfindung wird anhand einiger Figuren näher dargestellt:
- Figur 1:: Injektionssystem vor Aktivierung des Gasgenerators
- Figur 2:: Injektionssystem nach Aktivierung des Gasgenerators
- Figur 3:: Injektionssystem mit Membran zwischen erstem und zweitem Behältnis (vor Injektionsvorgang)
- Figur 4:: Wie Figur 3 nach Injektionsvorgang
- Figur 5:: Modul aus erstem Behältnis und Gasgenerator
- Figur 6:: Injektionssystem, bei der ein Modul gemäß Figur 5 in ein zweites Behältnis eingeschoben wird.

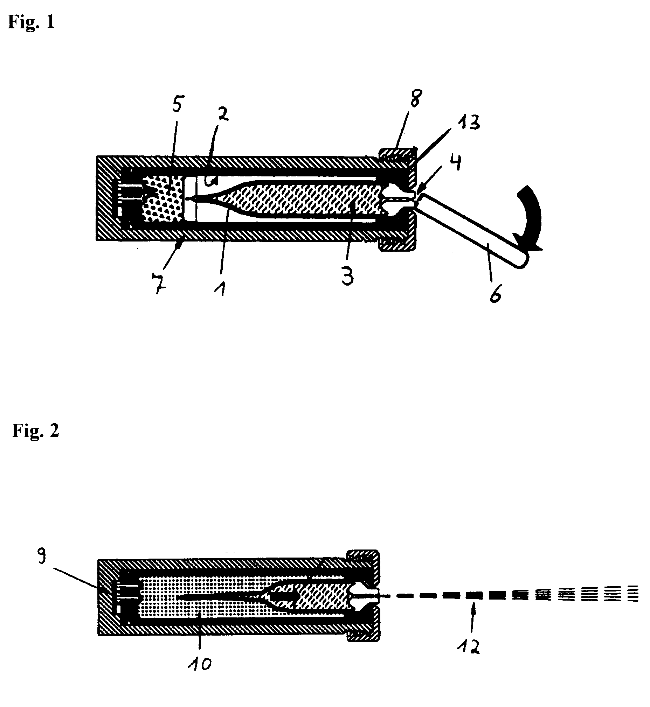

Die Figuren 1 und 2 zeigen eine erste Ausführungsform eines erfindungsgemäßen Injektionssystems vor und nach Aktivierung des Gasgenerators. In der Figur 1 ist das erste, zusammendrückbare Behältnis (1) zu erkennen, in dem sich eine Flüssigkeit (3) befindet.

Das erste Behältnis ist umgeben vom zweiten Behältnis (2), in dem sich auch der Gasgenerator (5) in Form eines Explosivstoffes befindet. Zwischen erstem und zweitem Behältnis befindet sich weiterhin ein Hohlraum, der mit Gas angefüllt oder evakuiert sein kann. Eine Füllung dieses Bereiches mit Gas ist dann vorteilhaft, wenn die Geschwindigkeit des Druckanstieges im zweiten Behaltnis verringert werden soll, während ein evakuierter Zwischenraum vorteilhaft ist, wenn ein möglichst geräuscharm arbeitendes System gewünscht ist. Das erste Behältnis besitzt an seinem vorderen Ende einen Kanal, der in eine Austrittsöffnung (4) mündet. Der Bereich, in dem sich der Kanal befindet, liegt im zweiten Bereich des ersten Behältnisses. In diesem Bereich ist das erste Behältnis vorzugsweise relativ stark ausgebildet, um eine sichere und insbesondere gasdichte Halterung zu ermöglichen. In seinem hinteren Ende, dem ersten Bereich, ist das erste Behaltnis zusammendrückbar gestaltet. Vorzugsweise ist dieser erste Bereich aus einem elastischen Kunststoff wie beispielsweise Polyethylen oder Polypropylen, gefertigt. Das erste Behältnis entspricht in seinem Aufbau Einweggefäßen für Augentropfen. Diese besitzen ebenfalls einen Bereich, der vom Benutzer zur Entleerung der Augentropfen zusammengedrückt wird, und eine Austrittsöffnung, die an das Auge herangeführt werden kann. Eine Herstellung des erfindungsgemäßen ersten Behältnisses ist daher für einen Fachmann aus dem Bereich wegwerfbarer Ampullen für Augentropfen bekannt.

Trotz der hohen, auf das erste Behältnis wirkenden Drücke, sind keine besonderen Vorkehrungen zur Erhöhung der mechanischen Stabilität notwendig. Dies liegt darin begründet, daß der entstehende Druck relativ gleichmäßig über die gesamte Außenhaut auf das erste Behältnis ausgeübt wird, so daß keine Scherkräfte auftreten, die zu einer starken mechanischen Belastung führen würden. Wie in der Figur 1 dargestellt, ist es vorteilhaft, wenn das erste Behältnis möglichst vollständig mit Flüssigkeit gefüllt ist. Zum einen wird durch eine Luftblase im ersten Behältnis ein Druckabfall durch die Kompressibilität des Gases verursacht und zweitens besteht die Gefahr, daß Gas in das Gewebe injiziert wird. Es hat sich jedoch herausgestellt, daß letztere Gefahr gering ist, da der auf Luft übertragbare Impuls aufgrund der geringen Dichte klein ist und somit normalerweise kein Durchdringen der Hautoberfläche erfolgt.

In der Figur 1 ist weiterhin ein Verschluß (6) dargestellt, der vor Benutzung des Systems die Austrittsöffnung (4) verschließt. Dieser Verschluß kann vorteilhaft über eine Sollbruchstelle mit dem ersten Behältnis verbunden sein, so daß ein Entfernen des Verschlusses durch Abknicken (wie dargestellt) oder Abdrehen erfolgen kann. Derartige Verschlüsse sind ebenfalls von Wegwerfampullen für Augentropfen bekannt. Diese Gefäße werden vorzugsweise als offenes Behältnis gespritzt, in das Flüssigkeit eingefüllt wird. Die Öffnung wird nachfolgend so verschweißt oder verschmolzen, daß ein Abdrehen des überstehenden Materialteils möglich ist. Bei der vorliegenden Erfindung ist es von Vorteil, wenn die Austrittsöffnung in ihrer Kontur und insbesondere die Form des Austrittskanals vorgegeben ist und nicht durch den Abdrehvorgang beeinflußt wird, wie dies bei den Ampullen für Augentropfen der Fall ist. Bei diesen wird durch den Abdrehvorgang des Verschlusses die Austrittsöffnung erst generiert, so daß die Form und der Innendurchmesser der Austrittsöffnung abhängig vom Abdrehvorgang ist. Bei erfindungsgemäß bevorzugten Behältnissen wird der Austrittskanal und die Austrittsöffnung hingegen bereits im Herstellungsprozeß, der in der Regel ein Spritzgußprozeß ist, geformt und die Sollbruchstelle, mit der der Verschluß an dem Behältnis befestigt ist, außerhalb des direkten Bereiches der Austrittsöffnung verlegt, so daß beim Abdrehen keine oder nur eine geringe Deformation der Austrittsöffnung erfolgt.

In der Figur 1 ist weiterhin zu erkennen, wie erstes und zweites Behältnis miteinander verbunden sind. Im dargestellten Fall befindet sich zwischen den Behältnissen ein Dichtungsmaterial (13), das den zweiten Bereich des ersten Behältnisses umgibt und das zweite Behaltnis gegen den Außenraum abschließt. Die Anordnung aus erstem und zweitem Behältnis befindet sich weiterhin in einer Stabilisierungshülle (7), die das zweite Behältnis umgibt und die durch eine Schraubkappe (8) verschlossen ist. Die Schraubkappe dient weiterhin dazu, die Dichtung (13) zu haltern.

In der Figur 2 ist zu erkennen, wie das erste Behältnis durch das im zweiten Behältnis freigesetzte Gas (10) zusammengedrückt wird und dabei einen Flüssigkeitsstrahl (12) ausstößt. In den Figuren 1 und 2 ist weiterhin eine Vorrichtung zur Aktivierung des Gasgenerators (5) zu erkennen, die aus dem zweiten Behältnis nach außen geführte elektrische Kontakte (9) aufweist, zwischen denen sich im Inneren des zweiten Behältnisses ein Glimmdraht befindet. Beim Hindurchleiten eines elektrischen Stromes durch den Glimmdraht erhitzt sich dieser so stark, daß er den Explosivstoff, der als Gasgenerator (5) dient, zündet.

Zur Durchführung eines Injektionsvorganges mit dem erfindungsgemäßen Injektionssystem wird die in den Figuren 1 und 2 dargestellte Vorrichtung mit der Austrittsöffnung auf eine Hautpartie aufgesetzt und der Gasgenerator aktiviert.

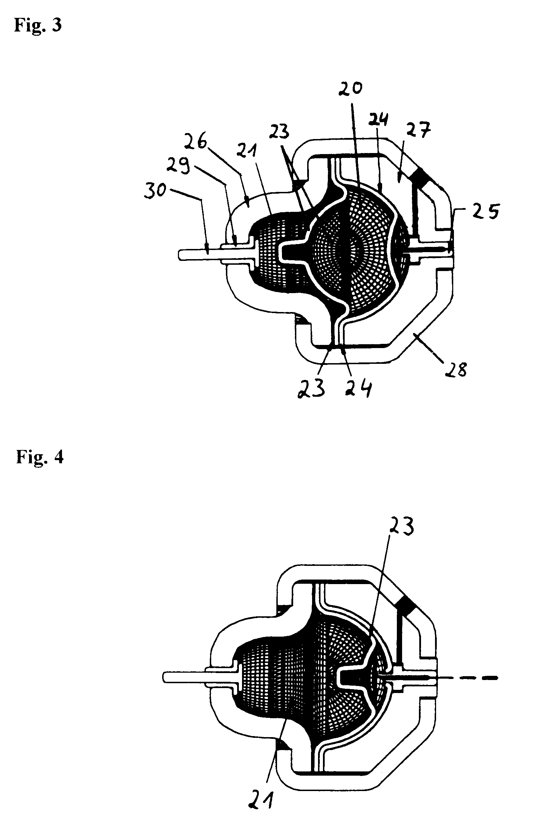

In den Figuren 3 und 4 ist eine zweite Ausführungsform eines erfindungsgemäßen Injektionssystems dargestellt. Zwischen erstem Behaltnis (20) und zweitem Behaltnis (21) befindet sich eine erste Membran (23), die bei Aktivierung des Gasgenerators im zweiten Behältnis von der in Figur 3 dargestellten Position in die in Figur 4 dargestellte Position bewegt wird. Diese Membran (23) kann aus einem Kunststoff, wie Polyethylen, oder auch einer Metallfolie, z. B. aus Aluminium, gefertigt sein. Auch bei dieser Ausführungsform des erfindungsgemäßen Injektionssystems werden gegeneinander verschiebbare Teile und die damit verbundenen Abdichtungsprobleme vermieden. In den Figuren 3 und 4 ist weiterhin eine zweite Membran (24) zu erkennen, die vorteilhafterweise eingesetzt werden kann Nach Aktivierung das Gasgenerators wird die erste Membran (23) in Richtung auf die Düse (25) bewegt und komprimiert die in dem ersten Behältnis (20) eingeschlossene Flüssigkeit, wodurch die zweite Membran (24) in Richtung auf die Düse (25) gedrängt wird, wobei sie von einer Hohlnadel, die in Richtung auf das erste Behältnis angeordnet ist, durchstochen wird. Nach Durchstechen der zweiten Membran wird die im ersten Behältnis befindliche Flüssigkeit durch die Düse herausgedrückt. Die in den Figuren 3 und 4 dargestellte Ausführungsform besitzt ein erstes und ein zweites Formteil (26, 27), die durch eine Spange (28) zusammengehalten werden. Zwischen den Formteilen sind erste und zweite Membran eingeklemmt. Das erste Formteil (26) besitzt eine Ausnehmung, in der sich eine Hülse (29) aus elektrisch isolierendem Material befindet, durch die ein elektrischer Kontakt (30) hindurchgeführt ist. Das erste Formteil (26) wird vorzugsweise aus elektrisch leitfähigem Material hergestellt, so daß durch Anlegen einer Spannung zwischen dem Formteil (26) und dem elektrischen Kontakt (30) eine Zündung des im zweiten Behältnis (21) befindlichen Explosivstoffes erfolgen kann. Bei Auswahl eines geeigneten Explosivstoffes kann auf einen Glimmdraht oder ähnliches verzichtet werden. Derartige Explosivstoffe enthalten in der Regel ein fein verteiltes Metall, wie beispielsweise Aluminium, durch das eine gewisse elektrische Leitfähigkeit des Explosivstoffes vermittelt wird.

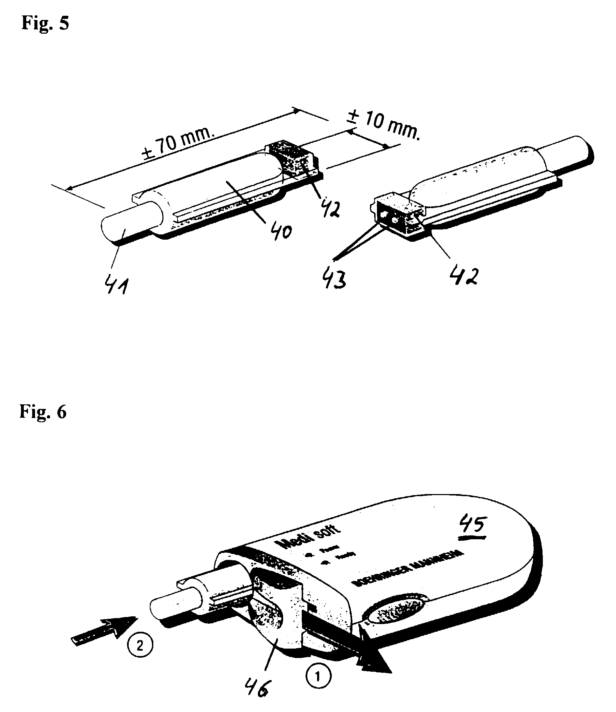

Figur 5 zeigt eine wegwerfbare Einheit für ein Injektionssystem, bei der erstes und zweites Behältnis getrennt voneinander vorliegen. In der Figur 5 ist eine Ausführungsform eines ersten Behältnisses (40) dargestellt, dessen Austrittsöffnung durch einen Zapfen (41) verschlossen ist. Es ist weiterhin der Gasgenerator (42) in Form eines Explosivstoffes zu erkennen, der sich außerhalb des ersten Behältnisses (40) befindet. In der ebenfalls dargestellten Rückansicht des Moduls sind elektrische Kontakte (43) zu erkennen, mit denen der Gasgenerator (42) gezündet werden kann.

In der Figur 6 ist eine Handhabungseinheit dargestellt, in die das in der Figur 5 dargestellte Modul eingeschoben werden kann. Hierzu besitzt die Handhabungseinheit (45) in ihrem Inneren einen Hohlkörper, in den das Modul aus Figur 5 eingeschoben werden kann. Hierzu wird zunächst der Schieber (46) zur Seite geschoben, so daß der Hohlkörper in der Handhabungseinheit geöffnet wird. Das Modul gemäß Figur 5 wird eingeschoben und der Schieber (46) verschlossen, wodurch eine Abdichtung des ersten und des zweiten Behältnisses gegeneinander erfolgt. Die Handhabungseinheit besitzt in ihrem Inneren elektrische Kontakte, die mit elektrischen Kontakten (43) des Moduls verbunden werden und über die durch eine Batterie eine Zündung erfolgt. Nach der Benutzung kann das entleerte erste Behältnis aus der Handhabungseinrichtung (45; durch Öffnung des Schiebers (46) entnommen und ggf. durch ein neues Modul ersetzt werden.

### Bezugszeichenliste

- (1): erstes, zusammendrückbares Behältnis
- (2): zweites Behältnis
- (3): Flüssigkeit
- (4): Austrittsöffnung
- (5): Gasgenerator
- (6): Verschluß
- (7): Stabilisierungshülle
- (8): Schraubkappe
- (9): elektrische Kontakte
- (10): Gas
- (12): Flüssigkeitsstrahl
- (13): Dichtungsmaterial
- (20): erstes Behältnis
- (21): zweites Behältnis
- (23): erste Membran
- (24): zweite Membran
- (25): Düse
- (26): erstes Formteil
- (27): zweites Formteil
- (28): Spange
- (29): Hülse
- (30): elektrischer Kontakt
- (40): erstes Behältnis
- (41): Verschlußzapfen
- (42): Gasgenerator
- (43): elektrische Kontakte
- (45): Handhabungseinheit
- (46): Schieber

## Patentansprüche

1. Transdermales Injektionssystem für Flüssigkeiten, beinhaltend
- ein erstes Behältnis, in dem sich die zu injizierende Flüssigkeit befindet und das zwei Bereiche besitzt, wobei der erste Bereich zusammendrückbar oder flexibel ist und der zweite Bereich mindestens eine Austrittsöffnung beinhaltet, durch die Flüssigkeit entweichen kann,
- ein zweites Behältnis, welches das erste Behältnis so umgibt, daß sich der zusammendrückbare Bereich zumindest teilweise innerhalb des zweiten Behältnisses befindet oder so an das erste Behältnis angrenzt, daß der flexible Bereich durch eine Druckänderung im zweiten Behältnis bewegt werden kann,
- einen aktivierbaren Gasgenerator, der sich innerhalb des zweiten Behältisses befindet und der bei Aktivierung einen Gasdruck innerhalb des zweiten Gefäßes erzeugt, wodurch das erste Behältnis zusammengedrückt und Flüssigkeit durch die mindestens eine Austrittsöffnung aus dem ersten Behältnis herausgepreßt wird.

2. Injektionssystem gemäß Anspruch 1, bei dem der zusammendrückbare Bereich des ersten Behältnisses aus einem elastischen Kunststoffhergestellt ist.

3. Transdermales Injektionssystem gemäß Anspruch 1, bei dem die Austrittsöffnung durch einen Verschluß verschlossen ist, der vor Benutzung des Systems entfernt wird.

4. Transdermales Injektionssystem gemäß Anspruch 3, bei dem der Verschluß mit dem zweiten Bereich über eine Sollbruchstelle verbunden ist.

5. Injektionssystem gemäß Anspruch 1, mit zwei oder mehr Austrittsöffnungen, die sich innerhalb des zweiten Bereiches befinden.

6. Injektionssystem gemäß Anspruch 1, bei dem der Gasgenerator ein Explosivstoff ist.

7. Injektionssystem gemäß Anspruch 1, das keine gegeneinander verschiebbaren Teile aufweist.

8. Injektionssystem beinhaltend ein einteilig ausgeführtes System gemaß Anspruch 1 sowie eine Handhabungseinheit, in die das einteilig ausgeführte System eingesetzt werden kann und die eine Vorrichtung zur Aktivierung des Gasgenerators beinhaltet.

9. Injektionssystem gemäß Anspruch 1, das zwei- oder mehrteilig ausgeführt ist und das erste Behältnis vom Benutzer in das zweite Behältnis eingesetzt und die entstehende Anordnung so verschlossen wird, daß ein im zweiten Gefäß entstehendes Gas nicht entweichen kann, sondern einen Druck auf den zusammendrückbaren Bereich des ersten Gefäßes ausgeübt wird.

10. Injektionssystem gemaß Anspruch 9, bei dem das zweite Behaltnis zu einer Handhabungseinheit gehört, in die das erste Behältnis eingesetzt wird und die weiterhin eine Vorrichtung zur Aktivierung des Gasgenerators beinhaltet.
